# EUROPÄISCHE PATENTSCHRIFT

(11) **EP 3 384 833 B1**
(45) Veröffentlichungstag und Bekanntmachung des Hinweises auf die Patenterteilung: **08.03.2023**
(21) Anmeldenummer: 17165537.6
(22) Anmeldetag: 07.04.2017
(51) Int. Cl.: A61B 5/00, A61B 5/055, G01R 33/48

(54) **INTERAKTIVE DATENERFASSUNG UND REKONSTRUKTION MITTELS EINER MAGNETRESONANZANLAGE**
INTERACTIVE DATA ACQUISITION AND RECONSTRUCTION USING A MAGNETIC RESONANCE SYSTEM
ENREGISTREMENT ET RECONSTRUCTION INTERACTIFS DE DONNÉES EN UTILISANT UN SYSTÈME DE RÉSONANCE MAGNÉTIQUE

(43) Veröffentlichungstag der Anmeldung: 10.10.2018
(73) Patentinhaber: Siemens Healthcare GmbH, 91052 Erlangen (DE); Centre Hospitalier Universitaire Vaudois, 1011 Lausanne (CH)
(72) Erfinder: CHAPTINEL, Jérôme, 1003 Lausanne (CH); KOBER, Tobias, 1007 Lausanne (CH); PICCINI, Davide, 1008 Prilly (CH); SPEIER, Peter, 91056 Erlangen (DE); STUBER, Matthias, 1032 Romanel-sur, Lausanne (CH); YERLY, Jérôme, 1637 Charmey (CH)

(56) Entgegenhaltungen:
- DE-A1-102014 202 513
- US-A1- 2006 100 499
- US-B1- 6 301 497
- JEROME CHAPTINEL ET AL.: "A Golden-Angle Acquisition Coupled with k-t Sparse SENSE Reconstruction for Fetal Self Retro-Gated Cine Cardiac MRI: an In Vivo Feasibility Study", JOURNAL OF THE INTERNATIONAL SOCIETY FOR MAGNETIC RESONANCE IN MEDICINE, ISMRM, Bd. 24, Nr. 459, 22. April 2016 (2016-04-22), XP040681502,
- MICHAEL S. HANSEN ET AL: "Retrospective reconstruction of high temporal resolution cine images from real-time MRI using iterative motion correction", MAGNETIC RESONANCE IN MEDICINE., Bd. 68, Nr. 3, 21. Dezember 2011 (2011-12-21), Seiten 741-750, XP055412031, US ISSN: 0740-3194, DOI: 10.1002/mrm.23284
- JÉRÔME YERLY ET AL: "Coronary endothelial function assessment using self-gated cardiac cine MRI and k - t sparse SENSE : Coronary Endothelial Function Assessment Using Self-Gated Cardiac Cine MRI", MAGNETIC RESONANCE IN MEDICINE., Bd. 76, Nr. 5, 24. November 2015 (2015-11-24), Seiten 1443-1454, XP055411936, US ISSN: 0740-3194, DOI: 10.1002/mrm.26050
- STEFANIE WINKELMANN ET AL: "An Optimal Radial Profile Order Based on the Golden Ratio for Time-Resolved MRI", IEEE TRANSACTIONS ON MEDICAL IMAGING, IEEE SERVICE CENTER, PISCATAWAY, NJ, US, Bd. 26, Nr. 1, 1. Januar 2007 (2007-01-01) , Seiten 68-76, XP011152669, ISSN: 0278-0062, DOI: 10.1109/TMI.2006.885337
- Brix Lau ET AL: "Feasibility of interactive magnetic resonance imaging of moving anatomy for clinical practice", CLINICAL PHYSIOLOGY AND FUNCTIONAL IMAGING, vol. 34, no. 1, 1 January 2014 (2014-01-01), pages 32-38, XP055894802, GB ISSN: 1475-0961, DOI: 10.1111/cpf.12061

## Beschreibung

Die vorliegende Erfindung betrifft eine interaktive Datenerfassung und Rekonstruktion mittels einer Magnetresonanzanlage, um insbesondere ein Herz eines Fötus im Mutterleib untersuchen zu können.

In "A Golden-Angle Acquisition Coupled with k-t-Sparse SENSE Reconstruction for Fetal Self Retro-Gated Cine Cardiac MRI: an In Vivo Feasability Study", von J. Chaptinel u.a., Journal of the International Society for Magnetic Resonance in Medicine, ISMRM, Nr. 459, 22. April 2016, XP040681502 wird eine MR-Bildgebung für eine retro-gated MR-Bildgebung des Herzens eines Fötus offenbart. Dazu werden Bilder einer hohen Zeitauflösung ("real-time eines") rekonstruiert, um daraus eine Art EKG-Signal abzuleiten. Anhand einer visuellen Inspektion der Bilder und des Signals können durch eine Bewegung des Fötus korrumpierte Daten von dem nachfolgenden Rekonstruktionsschritt ausgeschlossen werden.

Die DE 10 2014 202 513 A1 beschreibt das Erfassen von MR-Daten eines vorbestimmten Volumenabschnitts innerhalb eines Untersuchungsobjekts mit Hilfe einer Magnetresonanzanlage, wobei aus diesen MR-Daten mehrere MR-Bilder rekonstruiert werden. Jedes der MR-Bilder ist einem jeweiligen Zeitpunkt zugeordnet, wobei der zeitliche Abstand zwischen jeweils zwei zeitlich aufeinanderfolgenden Zeitpunkten nicht konstant ist.

In "Retrospective Reconstruction of High Temporal Resolution Cine Images from Real-Time MRI Using Iterative Motion Correction", von M. Hansen u.a., Magnetic Resonance in Medicine, Bd. 68, Nr. 3, 21. Dezember 2011, Seiten 741-750, XP055412031 wird eine retrospektive Rekonstruktion von MR-Bildern mittels einer iterativen Bewegungskorrektur offenbart. Dabei können die Daten mit verschiedenen zeitlichen Auflösungen rekonstruiert werden.

In "Coronary Endothelial Function Assessment Using Self-Gated Cardiac Cine MRI and k-t Sparse SENSE", von J. Yerly u.a., Magnetic Resonance in Medicine, Bd. 76, Nr. 5, 24. November 2015, Seiten 1443-1454, XP055411936 wird beschrieben, wie MR-Daten mit einem auf dem goldenen Winkel basierenden Erfassungsschema erfasst werden. Anhand von MR-Bildern mit geringer Auflösung, die aus diesen Daten rekonstruiert werden, kann ein "Self-Gating"-Signal extrahiert werden.

In "An Optimal Radial Profile Order Based on the Golden Ratio for Time-Resolved MRI", von S. Winkelmann u.a., IEEE Transactions on Medical Imaging, Bd. 26, Nr. 1, 1. Januar 2007, Seiten 68-76, XP011152669 wird eine MR-Bildgebung beschrieben, bei der Trajektorien um einen Winkel beabstandet sind, der auf dem goldenen Schnitt basiert.

US 2006/100499 offenbart die Rekonstruktion und die Darstellung von MRI Bildern in Echtzeit.

Eine angeborene Herzerkrankung tritt bei ungefähr 4 bis 13 Fällen von 1000 Lebendgeburten auf und ist aktuell der wichtigste Grund für die Kindersterblichkeit. Zur Erkennung einer angeborene Herzerkrankung ist aktuell eine Echokardiographie des Fötus das Mittel der Wahl, da damit eine sichere, einfach durchzuführende und preisgünstige Untersuchungsmethode bereitsteht, mit welcher Bilder in Echtzeit erzeugt werden können und schwere angeborene Herzerkrankungen sicher erkannt werden können. Jedoch ist eine Echokardiographie des Fötus stark vom untersuchenden Mediziner abhängig und ist bisweilen durch akustische Fenster, Schwierigkeiten bei der Bildgebung des distalen Gefäßsystems, der Position des Fötus, der Fettleibigkeit der Mutter oder von Narben im Bauchraum von vorhergehenden Operationen beschränkt.

Da die vorab beschriebenen Faktoren die Qualität einer MR-Bildgebung nahezu nicht beeinflussen, wird nach dem Stand der Technik eine MR-Bildgebung im zweiten und dritten Trimester als komplementäres Untersuchungsverfahren eingesetzt, wenn beispielsweise die Diagnose mittels der Echokardiographie nicht eindeutig ist. Allerdings basiert die MR-Bildgebung prinzipiell auf der Annahme, dass sich das zu untersuchende Objekt während der Datenerfassung nicht bewegt. Genauer gesagt sollten alle Daten, welche während einer einzigen Datenerfassung gesammelt werden, räumlich konsistent zueinander sein. Bei der MR-Bildgebung des Herzens eines Fötus ergeben sich daher folgende Anforderungen bzw. Probleme:
- Es ist nicht möglich, ein EKG-Signal des Fötus zu erfassen, um die Datenerfassung mit dem Herzschlag des Fötus zu synchronisieren, wie es bei der MR-Bildgebung des Herzens eines Erwachsenen nach dem Stand der Technik üblich ist.
- Aufgrund der unvorhersehbaren und spontanen Bewegung des Fötus ist die Planung der Datenerfassung sehr schwierig. Häufig werden entsprechende Bewegungen des Fötus erst in den rekonstruierten Bildern erkannt.

Die vorliegende Erfindung stellt sich die Aufgabe, eine MR-Bildgebung eines Herzens eines Fötus bereitzustellen, wobei die vorab beschriebenen Probleme gelöst werden.

Erfindungsgemäß wird diese Aufgabe durch ein Verfahren zur interaktiven Erfassung von Daten nach Anspruch 1, durch eine Magnetresonanzanlage nach Anspruch 11, durch ein Computerprogrammprodukt nach Anspruch 13 und durch einen elektronisch lesbaren Datenträger nach Anspruch 14 gelöst. Die abhängigen Ansprüche beschreiben bevorzugte und vorteilhafte Ausführungen der vorliegenden Erfindung.

Im Rahmen der vorliegenden Erfindung wird ein Verfahren zur interaktiven Erfassung von MR-Daten eines Untersuchungsobjekts mittels einer Magnetresonanzanlage bereitgestellt. Dabei umfasst das erfindungsgemäße Verfahren folgende Schritte:
- Erfassen der MR-Daten in einem Volumenabschnitt des Untersuchungsobjekts mit der Magnetresonanzanlage.
- Automatisches Rekonstruieren von MR-Bildern anhand der erfassten MR-Daten und Darstellen der MR-Bilder in Echtzeit. Die Darstellung der MR-Bilder in Echtzeit bedeutet dabei, dass die MR-Bilder dargestellt werden, während die Datenerfassung (für folgende Bilder) noch läuft. Beispielsweise kann die Datenerfassung von einem MR-Bild eine Zeitspanne von 0,5 s und die Rekonstruktion des MR-Bildes weitere 0,5 s dauern, so dass die MR-Bilder ungefähr 1 s nach Beginn der Datenerfassung der MR-Daten des jeweiligen MR-Bildes dargestellt werden.
- Bestimmen einer Zeitspanne, in welcher eine vorbestimmte Bedingung in den dargestellten MR-Bildern erfüllt ist. In diesem Schritt kann anhand der dargestellten MR-Bilder überprüft werden, ob die bestimmte Bedingung die Zeitspanne lang eingehalten wird. Damit kann vorteilhafterweise quasi indirekt auch überprüft werden, ob die vorbestimmte Bedingung die Zeitspanne lang während der Datenerfassung erfüllt war.
- Automatisches Rekonstruieren von Qualitäts-MR-Bildern anhand derjenigen MR-Daten, welche während der Zeitspanne erfasst wurden. Dabei ist die zeitliche Auflösung der Qualitäts-MR-Bilder höher als die zeitliche Auflösung der MR-Bilder, welche in Echtzeit dargestellt werden. Die automatische Rekonstruktion der Qualitäts-MR-Bilder findet dabei insbesondere offline statt, da die Qualitäts-MR-Bilder in der Regel nicht in Echtzeit dargestellt werden. Darüber hinaus kann auch die örtliche Auflösung der Qualitäts-MR-Bilder höher sein als die örtliche Auflösung der in Echtzeit dargestellten MR-Bilder.

Indem anhand der in Echtzeit dargestellten MR-Bilder überprüft wird, ob die vorbestimmte Bedingung erfüllt ist, ist vorteilhafterweise sichergestellt, dass diese Bedingung auch für die MR-Daten erfüllt ist, aus welchen die Qualitäts-MR-Bilder rekonstruiert werden. Die Verwendung derselben MR-Daten sowohl zur Rekonstruktion der in Echtzeit dargestellten MR-Bilder als auch zur Rekonstruktion der Qualitäts-MR-Bilder weist somit zum einen den Vorteil auf, dass dieselben MR-Daten zur Rekonstruktion von zwei verschiedenen Arten von MR-Bildern eingesetzt werden. Zum anderen weist die Verwendung derselben MR-Daten für die MR-Bilder und für die Qualitäts-MR-Bilder den Vorteil auf, dass anhand der in Echtzeit dargestellten MR-Bilder überprüft werden kann, dass die vorbestimmte Bedingung für die MR-Daten erfüllt ist.

Insbesondere handelt es sich bei dem Untersuchungsobjekt um ein Herz eines lebenden Fötus, welcher sich innerhalb des Mutterleibs befindet.

Damit kann die vorliegende Erfindung vorteilhafterweise zur Untersuchung eines angeborenen Herzleidens bei einem Fötus anstelle einer fetalen Echokardiographie eingesetzt werden.

Bei der vorbestimmten Bedingung kann es sich um eine Bewegung des Fötus innerhalb der Zeitspanne handeln. Mit anderen Worten ist gemäß dieser bevorzugten Ausführungsform die vorbestimmte Bedingung in den dargestellten MR-Bildern dann erfüllt, wenn innerhalb dieser Zeitspanne keine Bewegung des Fötus erkannt wird.

Diese Ausführungsform bietet den Vorteil, dass anhand der in Echtzeit dargestellten MR-Bilder sichergestellt wird, dass sich der Fötus innerhalb der vorbestimmten Zeitspanne nicht bewegt. Dadurch ist wiederum vorteilhafterweise sichergestellt, dass die Rekonstruktion der Qualitäts-MR-Bilder nicht durch eine Bewegung des Fötus negativ beeinflusst wird.

Gemäß einer weiteren erfindungsgemäßen Ausführungsform erfolgt das Erfassen der MR-Daten mittels einer zweidimensionalen radialen Datenerfassung, bei welcher die MR-Daten entlang von radial verlaufenden Trajektorien erfasst werden. Der Winkel, welcher zwischen zwei zeitlich direkt aufeinanderfolgenden radial verlaufenden Trajektorien auftritt, entspricht dabei dem goldenen Winkel.

Unter dem goldenen Winkel wird dabei in der Regel der kleinere goldene Winkel verstanden, welcher einen Winkel von 180° im goldenen Schnitt teilt. Es ist allerdings erfindungsgemäß auch möglich, dass der größere goldene Winkel eingesetzt wird, welcher den Winkel von 180° im goldenen Schnitt teilt und zusammen mit dem kleineren goldenen Winkel 180° ergibt. Darüber hinaus kann erfindungsgemäß auch der kleinere oder größere goldene Winkel eingesetzt werden, welche jeweils einen Vollkreis (360°) im goldenen Schnitt teilen.

Den Winkel zwischen jeweils zwei zeitlich aufeinanderfolgenden radial verlaufenden Trajektorien gemäß dem goldenen Winkel zu wählen, minimiert vorteilhafterweise Artefakte aufgrund von schnell schaltenden Gradienten (z.B. Wirbelstrom-Artefakte).

Gemäß einer Variante der vorab beschriebenen Ausführungsform werden die MR-Daten erfasst, indem dieselbe Menge der radial verlaufenden Trajektorien mehrfach erfasst wird. Dabei entspricht die Anzahl der Trajektorien dieser Menge einer hohen Zahl der Fibonacci-Folge (z.B. 987 oder größer). Mit anderen Worten wird die Menge der Trajektorien ein erstes Mal erfasst, wobei zeitlich anschließend die Menge der Trajektorien ein zweites Mal erfasst wird, usw..

Indem die Anzahl der Trajektorien einer hohen Zahl der Fibonacci-Folge entspricht, ist sichergestellt, dass der Winkel der letzten Trajektorie bei der n-ten Datenerfassung der Menge der Trajektorien ziemlich genau bei 0° liegt. Wenn dann die erste Trajektorie bei der (n+1)-ten Datenerfassung der Menge der Trajektorien den goldenen Winkel zu einer bei 0° liegenden Ausgangsgeraden aufweist, entspricht auch der Winkel zwischen der letzten Trajektorie der n-ten Datenerfassung und der ersten Trajektorie der (n+1)-ten Datenerfassung nahezu dem goldenen Winkel.

Indem die Anzahl der Trajektorien also der hohen Zahl der Fibonacci-Folge entspricht, entspricht die erfindungsgemäße Datenerfassung quasi einer Datenerfassung, bei welcher jede folgende Trajektorie jeweils den goldenen Winkel zur vorherigen Trajektorie aufweist, obwohl die Datenerfassung in mehrere Abschnitte unterteilt ist, wobei in jedem Abschnitt jeweils die MR-Daten entlang der Menge der Trajektorien erfasst werden.

Wenn die Anzahl der Trajektorien der Menge beispielsweise der Zahl 6765 der Fibonacci-Folge entspricht, können für jeden Abschnitt der Datenerfassung beispielsweise die MR-Daten für 55 MR-Bilder erfasst werden, wobei die MR-Daten für jedes MR-Bild entlang von 123 Trajektorien erfasst werden (55 * 123 = 6765) .

Erfindungsgemäß ist es allerdings auch möglich, dass die Daten mittels einer radialen Datenerfassung erfasst werden, wobei die Trajektorien eine vorbestimmte pseudo-zufällige Verteilung aufweisen. Auch der Einsatz von vorbestimmten pseudozufällig verteilten spiralförmig verlaufenden Trajektorien ist möglich.

Die (pseudo-) zufällige Verteilung der Trajektorien verkleinert vorteilhafterweise die Artefakte in den rekonstruierten MR-Bildern und Qualitäts-MR-Bildern.

Da der Verlauf der Trajektorien bereits bekannt ist, kann die Rekonstruktion der MR-Bilder und/oder der Qualitäts-MR-Bilder anhand von vorab berechneten (z.B. bereits zur Datenerfassung vorliegenden) Parametern erfolgen.

Da die Trajektorien und damit die K-Raum-Punkte für welche die MR-Daten erfasst werden, bereits im Vorfeld bekannt sind, können die entsprechenden Parameter für die Rekonstruktion ebenfalls bereits im Vorfeld berechnet werden. Dadurch kann vorteilhafterweise die Rekonstruktion der MR-Bilder und/oder der Qualitäts-MR-Bilder beschleunigt werden.

Das Erfassen der MR-Daten in mehreren Abschnitten von jeweils m Trajektorien, welche jeweils im goldenen Winkel beabstandet sind, wobei die Anzahl m einer hohen Zahl der Fibonacci-Folge entspricht, weist demnach folgende Vorteile auf:
- Zum einen entspricht die Datenerfassung quasi einer reinen Datenerfassung mit dem goldenen Winkel, bei welcher für jede Trajektorie (bis auf die erste) gilt, dass sie exakt den goldenen Winkel zu ihrer Vorgänger-Trajektorie aufweist.
- Zum anderen können die Parameter zur Rekonstruktion dennoch vorberechnet werden, um die Rekonstruktion zu beschleunigen.

Zur Rekonstruktion der Qualitäts-MR-Bilder wird insbesondere eine K-T-Sparse-SENSE ("SENSivity Encoding")-Rekonstruktion durchgeführt. Dabei bedeutet "K-T", dass die MR-Daten zu verschiedenen Zeitpunkten ("T") im K-Raum ("K") erfasst werden. "Sparse" bedeutet, dass bei der Datenerfassung der K-Raum nicht vollständig, sondern nur spärlich (unvollständig) erfasst wird.

Insbesondere umfasst die Rekonstruktion der Qualitäts-MR-Bilder folgende drei Schritte:
- Rekonstruieren von MR-Zwischenbildern aus den MR-Daten. Diese Rekonstruktion kann beispielsweise eine direkte K-T-Sparse-SENSE-Rekonstruktion sein.
- Ableiten einer Information über die Herzaktivität des Fötus aus den MR-Zwischenbildern. In diesem Schritt wird automatisch anhand der MR-Zwischenbilder eine einem EKG-Signal entsprechende Information über den Herzschlag des Fötus abgeleitet. Die MR-Zwischenbilder weisen eine geringere Bildqualität auf, als die Qualitäts-MR-Bilder. Allerdings ist die Bildqualität (insbesondere die zeitliche Auflösung) der MR-Zwischenbilder gut genug, um anhand der MR-Zwischenbilder die Information über die Herzaktivität ableiten zu können.
- Rekonstruieren der Qualitäts-MR-Bilder abhängig von der Information über die Herzaktivität des Fötus. Anhand der Information über die Herzaktivität kann zu jedem Zeitpunkt abgeleitet werden, in welcher Herzschlagphase sich das Herz des Fötus befindet. Indem diese Information über die jeweilige Herzschlagphase bei der Rekonstruktion der Qualitäts-MR-Bilder berücksichtigt wird, kann die Qualität der Qualitäts-MR-Bilder vorteilhafterweise verbessert werden. Auch bei der Rekonstruktion der Qualitäts-MR-Bilder kann eine K-T-Sparse-SENSE-Rekonstruktion eingesetzt werden, welche anhand der Information über die Herzaktivität des Fötus geführt wird.

Eine Rekonstruktion anhand der vorab skizzierten drei Schritte kann auch als dreistufige Rekonstruktion bezeichnet werden.

Gemäß einer weiteren erfindungsgemäßen Ausführungsform werden die MR-Daten in einer Schicht innerhalb des Untersuchungsobjekts erfasst, so dass die in Echtzeit rekonstruierten und dargestellten MR-Bilder Abbildern dieser Schicht entsprechen. Anhand dieser MR-Bilder kann dann überprüft werden, ob die Lage der Schicht für die durchzuführende Untersuchung geeignet ist. Bei einer Untersuchung der Herzaktivität eines Fötus im Mutterleib kann beispielsweise überprüft werden, ob die Schicht korrekt im Herzen des Fötus angeordnet ist. Falls dies nicht der Fall ist, kann abhängig von den in Echtzeit dargestellten MR-Bildern die Lage der Schicht geändert werden. Dazu ermittelt beispielsweise der die Magnetresonanzanlage bedienende Mediziner anhand der dargestellten MR-Bilder und gegebenenfalls anhand von weiteren mit einem Localizer erstellten MR-Bildern, in welcher Weise die Lage der Schicht zu verändern ist. Daher wird eine Information zur Änderung der Lage der Schicht mit Hilfe der in Echtzeit dargestellten MR-Bilder erfasst. Abhängig von dieser Information wird dann die Lage der Schicht entsprechend geändert und im Folgenden die MR-Daten für diese Schicht (in der neuen Lage) erfasst.

Dieses Vorgehen (Erfassen von MR-Daten der Schicht (in der jeweils aktuellen Lage), Darstellen der rekonstruierten MR-Bilder in Echtzeit, Überprüfen der Lage der Schicht und Ändern der Lage der Schicht) kann so oft durchgeführt werden, bis die Lage der Schicht für die durchzuführende Untersuchung geeignet ist.

Diese Ausführungsform ermöglicht beispielsweise, dass eine Bedienperson (z.B. der die Magnetresonanzanlage bedienende Mediziner) z.B. anhand von MR-Bildern eines Localizers das Herz des Fötus lokalisiert, um dann eine Schicht, von welcher MR-Daten zu erfassen sind, möglichst gut bezüglich des Herzens des Fötus anzuordnen. Anhand der in Echtzeit dargestellten erfindungsgemäßen MR-Bilder kann dann die Lage der Schicht kontrolliert und Informationen oder Anweisungen der Bedienperson zur Änderung dieser Lage erfasst werden, um dann abhängig von diesen Informationen oder Anweisungen die Lage der Schicht einzustellen.

Im Rahmen der vorliegenden Erfindung wird auch eine Magnetresonanzanlage bereitgestellt, welche ausgestaltet ist, um interaktiv Daten eines Untersuchungsobjekts zu erfassen. Die Magnetresonanzanlage umfasst eine HF-Steuereinheit, eine Gradientensteuereinheit und eine Bildsequenzsteuerung, welche ausgestaltet sind, um die Daten des Untersuchungsobjekts zu erfassen. Darüber hinaus ist eine Recheneinheit der Magnetresonanzanlage ausgestaltet, um MR-Bilder anhand der MR-Daten in Echtzeit zu rekonstruieren. Eine Anzeigeeinheit der Magnetresonanzanlage ist ausgestaltet, um die rekonstruierten Bilder darzustellen. Mit einer Eingabeeinheit der Magnetresonanzanlage wird eine Zeitspanne bestimmt, in welcher eine bestimmte Bedingung in den Bildern erfüllt ist. Die Recheneinheit ist zusätzlich ausgestaltet, um anhand der MR-Daten, welche innerhalb der Zeitspanne erfasst wurden, Qualitäts-MR-Bilder zu rekonstruieren. Dabei ist die zeitliche Auflösung bei der Rekonstruktion der Qualitäts-MR-Bilder höher als die zeitliche Auflösung bei der Rekonstruktion der MR-Bilder.

Die Vorteile der erfindungsgemäßen Magnetresonanzanlage entsprechen dabei im Wesentlichen den Vorteilen des erfindungsgemäßen Verfahrens, welche vorab im Detail ausgeführt worden sind, so dass hier auf eine Wiederholung verzichtet wird.

Des Weiteren beschreibt die vorliegende Erfindung ein Computerprogrammprodukt, insbesondere ein Computerprogramm oder eine Software, welche man in einen Speicher einer programmierbaren Steuerung bzw. einer Recheneinheit einer Magnetresonanzanlage laden kann. Mit diesem Computerprogrammprodukt können alle oder verschiedene vorab beschriebene Ausführungsformen des erfindungsgemäßen Verfahrens ausgeführt werden, wenn das Computerprogrammprodukt in der Steuerung oder Steuereinrichtung der Magnetresonanzanlage läuft. Dabei benötigt das Computerprogrammprodukt eventuell Programmmittel, z.B. Bibliotheken und Hilfsfunktionen, um die entsprechenden Ausführungsformen des Verfahrens zu realisieren. Mit anderen Worten soll mit dem auf das Computerprogrammprodukt gerichteten Anspruch insbesondere ein Computerprogramm oder eine Software unter Schutz gestellt werden, mit welcher eine der oben beschriebenen Ausführungsformen des erfindungsgemäßen Verfahrens ausgeführt werden kann bzw. welche diese Ausführungsform ausführt. Dabei kann es sich bei der Software um einen Quellcode (z.B. C++), der noch compiliert (übersetzt) und gebunden oder der nur interpretiert werden muss, oder um einen ausführbaren Softwarecode handeln, der zur Ausführung nur noch in die entsprechende Recheneinheit bzw. Steuereinrichtung zu laden ist.

Schließlich offenbart die vorliegende Erfindung einen elektronisch lesbaren Datenträger, z.B. eine DVD, ein Magnetband, eine Festplatte oder einen USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuereinrichtung bzw. Recheneinheit einer Magnetresonanzanlage gespeichert werden, können alle erfindungsgemäßen Ausführungsformen des vorab beschriebenen Verfahrens durchgeführt werden.

Im Folgenden wird die vorliegende Erfindung anhand bevorzugter erfindungsgemäßer Ausführungsformen mit Bezug zu den beigefügten Figuren im Detail beschrieben.

Fig. 1 zeigt schematisch eine Magnetresonanzanlage, mit welcher erfindungsgemäß interaktiv MR-Daten eines Untersuchungsobjekts erfasst werden können.

In Fig. 2 ist schematisch dargestellt, wie erfindungsgemäß die Lage einer Schicht auch anhand der in Echtzeit dargestellten MR-Bilder geändert werden kann.

In Fig. 3 ist der Flussplan einer Ausführungsform des erfindungsgemäßen Verfahrens dargestellt.

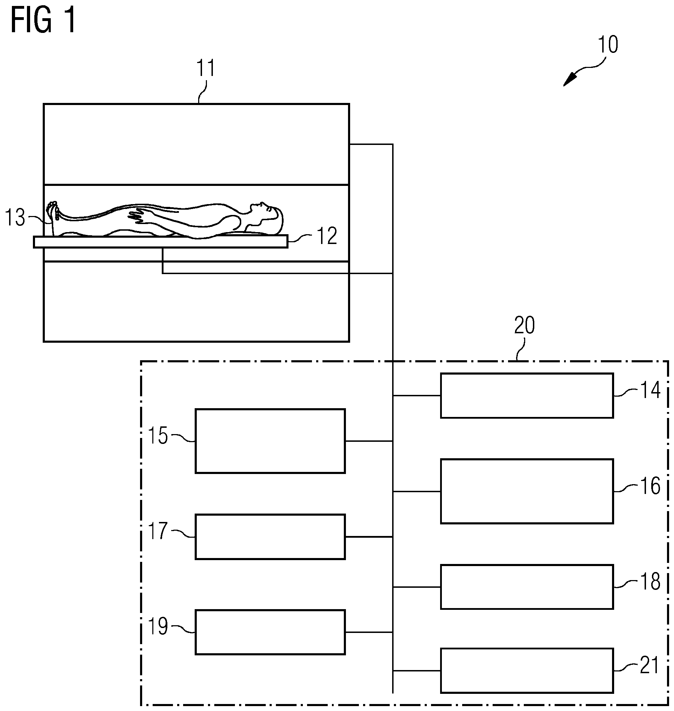

In Bezug auf Fig. 1 wird eine Magnetresonanzanlage erläutert, mit der, wie nachfolgend erläutert wird, MR-Daten eines Untersuchungsobjekts erfasst werden können. Die Magnetresonanzanlage 10 weist einen Magneten 11 zur Erzeugung eines Polarisationsfelds B0 auf, wobei eine auf einer Liege 12 angeordnete Untersuchungsperson 13 in den Magneten 11 gefahren wird, um dort ortskodierte Magnetresonanzsignale aus der Untersuchungsperson 13 und insbesondere aus einem Herzen eines in der Untersuchungsperson 13 befindlichen Fötus aufzunehmen. Die zur Signalaufnahme verwendeten Spulen, wie eine Ganzkörperspule oder Lokalspulen, sind aus Übersichtlichkeitsgründen nicht dargestellt. Die Erfindung kann bei der sogenannten parallelen Bildgebung Anwendung finden, bei der die MR-Signale gleichzeitig mit mehreren Lokalspulen, einem Spulenarray von Lokalspulen, aufgenommen werden. Durch Einstrahlen von Hochfrequenzpulsen und Schalten von Magnetfeldgradienten kann die durch das Polarisationsfeld B0 erzeugte Magnetisierung aus der Gleichgewichtslage ausgelenkt und ortskodiert werden, und die sich ergebende Magnetisierung wird von den Empfangsspulen detektiert. Wie durch Einstrahlen der HF-Pulse und durch Schalten von Magnetfeldgradienten in verschiedenen Kombinationen und Reihenfolgen MR-Bilder erzeugt werden können, ist dem Fachmann grundsätzlich bekannt und wird hier nicht näher erläutert.

Die Magnetresonanzanlage 10 weist weiterhin eine Steuereinheit 20 auf, die zur Steuerung der Magnetresonanzanlage 10 verwendet werden kann. Die Steuerung 20 weist eine Gradientensteuereinheit 15 zur Steuerung und Schaltung der notwendigen Magnetfeldgradienten auf. Eine HF-Steuereinheit 14 ist für die Steuerung und Generierung der HF-Pulse zur Auslenkung der Magnetisierung vorgesehen. Eine Bildsequenzsteuerung 16 steuert die Abfolge der Magnetfeldgradienten und HF-Pulse und damit indirekt die Gradientensteuereinheit 15 und die HF-Steuereinheit 14. Über eine Eingabeeinheit 17 kann eine Bedienperson die Magnetresonanzanlage steuern und Informationen eingeben, und auf einer Anzeigeeinheit 18 können MR-Bilder und sonstige zur Steuerung notwendigen Informationen angezeigt werden. Eine Recheneinheit 19 mit mindestens einer Prozessoreinheit (nicht gezeigt) ist vorgesehen zur Steuerung der verschiedenen Einheiten in der Steuereinheit 20. Weiterhin ist eine Speichereinheit 21 vorgesehen, in der beispielsweise Programmmodule bzw. Programme abgespeichert sein können, die, wenn sie von der Recheneinheit 19 bzw. ihrer Prozessoreinheit ausgeführt werden, den Ablauf der Magnetresonanzanlage steuern können. Die Recheneinheit 19 ist, wie nachfolgend erläutert wird, ausgebildet, um aus den erfassten MR-Daten die MR-Bilder und die Qualitäts-MR-Bilder erfindungsgemäß zu berechnen.

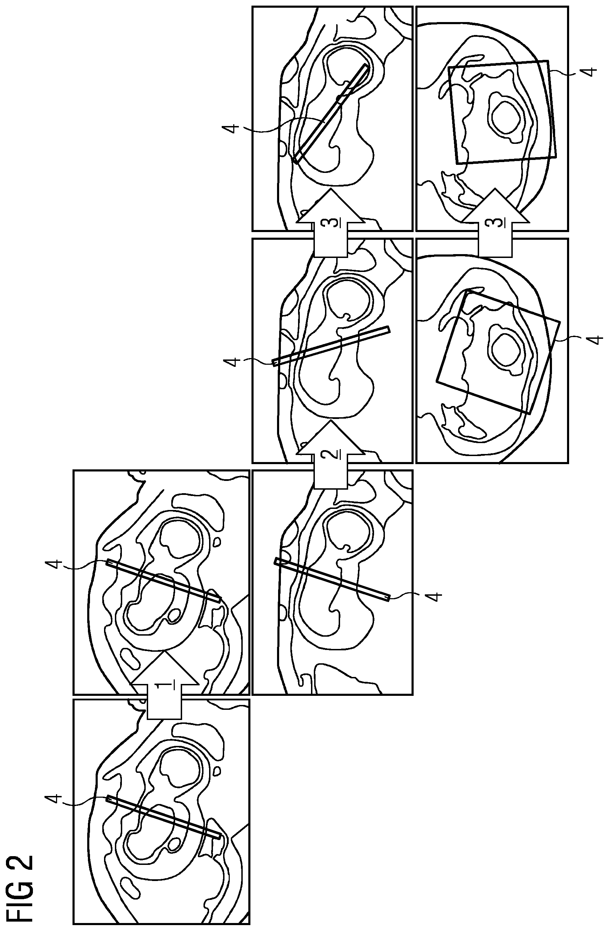

In Fig. 2 ist schematisch dargestellt, wie die Lage einer Schicht, in welcher MR-Daten zu erfassen sind, erfindungsgemäß geändert werden kann.

Anhand von Übersichtsbildern platziert eine Bedienperson (in der Regel ein die Magnetresonanzanlage bedienender Mediziner) eine Schicht 4 im gewünschten Volumenabschnitt des Untersuchungsobjekts (z.B. im Herzen des Fötus im Mutterleib). Indem MR-Bilder (nicht dargestellt), welche ausgehend von den in der Schicht 4 erfassten MR-Daten rekonstruiert werden und in Echtzeit dargestellt werden, kann die Bedienperson sehr rasch überprüfen, ob die Lage der Schicht 4 korrekt ist. Falls die Bedienperson mit der aktuellen Lage der Schicht nicht zufrieden ist, können erfindungsgemäß Anweisungen oder Informationen erfasst werden, um die Lage abhängig von diesen Anweisungen oder Informationen zu ändern.

In Fig. 2 sind mögliche Änderungen der Lage der Schicht dargestellt. Mit den Bildabschnitten in Fig. 2, welche über den mit dem Bezugszeichen 1 bezeichneten Pfeil verbunden sind, wird eine einfache Verschiebung der Schicht 4 ohne Änderung des Richtungsvektors der Schicht 4 dargestellt. Dagegen wird mit den Bildabschnitten, welche über den mit dem Bezugszeichen 2 bezeichneten Pfeil verbunden sind, eine Drehung der Schicht 4 dargestellt, wobei der Richtungsvektor der Schicht in der dargestellten Ebene verbleibt. Schließlich stellen die Bildabschnitte, welche über die mit den Bezugszeichen 3 bezeichneten Pfeile verbunden sind, eine Verlagerung der Schicht 4 dar, bei welcher die Schicht 4 sowohl verschoben als auch gekippt wird.

Sobald sich die Lage der Schicht 4 beispielsweise durch eine der in Fig. 2 dargestellten Änderungen verändert hat, werden die nächsten MR-Daten für die jeweils aktuelle Schicht 4 erfasst und in Echtzeit in ein MR-Bild rekonstruiert, welches dargestellt wird. Dadurch bekommt der Mediziner (Benutzer der Magnetresonanzanlage) sofort eine Rückmeldung, ob die aktuelle Lage der Schicht 4 seinen Vorstellungen entspricht. Falls dies nicht der Fall ist, kann er die Lage der Schicht 4 jederzeit erneut entsprechend ändern bzw. korrigieren.

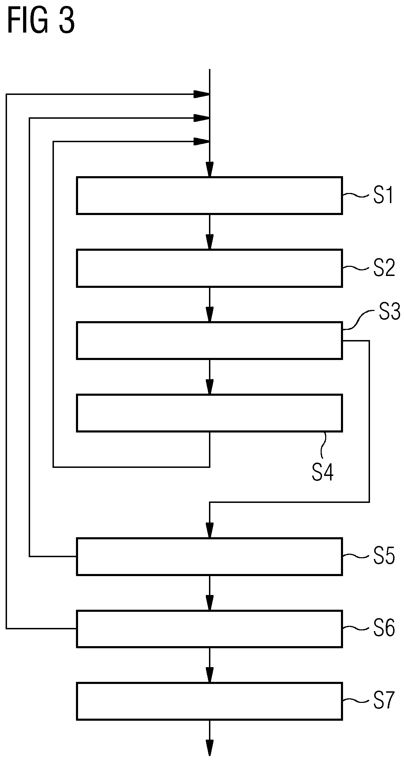

In Fig. 3 ist der Flussplan einer Ausführungsform des erfindungsgemäßen Verfahrens dargestellt.

Im Schritt S1 werden MR-Daten erfasst. Ausgehend von diesen MR-Daten werden im Schritt S2 MR-Bilder derart rekonstruiert, dass sie im selben Schritt S2 mit einer Verzögerung unterhalb der Bildwiederholungsrate (also quasi in Echtzeit) dargestellt werden.

Im Schritt S3 überprüft der Mediziner (Benutzer der Magnetresonanzanlage) anhand der dargestellten MR-Bilder, ob die Lage der Schicht 4 seinen Wünschen entspricht (also z.B. durch das Herz des Fötus verläuft). Falls dies nicht der Fall ist, verzweigt das Verfahren zum Schritt S4, in welchem der Mediziner die Schichtlage gemäß seinen Vorstellungen ändert, wie es beispielhaft in Fig. 2 dargestellt ist. In diesem Fall springt das Verfahren vom Schritt S4 zurück zum Schritt S1.

Falls der Mediziner im Schritt S3 mit der aktuellen Lage der Schicht 4 zufrieden ist, verzweigt das erfindungsgemäße Verfahren vom Schritt S3 zum Schritt S5. In diesem Schritt S5 wird anhand der dargestellten MR-Bilder der Schicht 4 überprüft, ob sich das Untersuchungsobjekt (in diesem Fall insbesondere der Fötus) ausreichend ruhig verhält. Dazu wird bei einer Untersuchung des Fötus die Mutter gebeten, ihren Atem anzuhalten. Wenn trotzdem anhand der dargestellten MR-Bilder erkannt wird, dass eine störende Bewegung des Untersuchungsobjekts (z.B. Fötus) vorliegt, springt das Verfahren zurück zum Schritt S1. Wenn dagegen während einer vorbestimmten Zeitspanne bei einer Kontrolle durch die dargestellten MR-Bilder keine störende Bewegung des Untersuchungsobjekts erkannt wird, verzweigt das erfindungsgemäße Verfahren zum Schritt S6.

Im Schritt S6 wird überprüft, ob die Menge der MR-Daten, welche ohne störende Bewegung erfasst wurde, ausreichend groß ist, um beispielsweise das Herz des Fötus auf eine angeborene Erkrankung zu untersuchen. Wenn dies der Fall ist, verzweigt das Verfahren zum Schritt S7, andernfalls zurück zum Schritt S1. Durch den Rücksprung zu Schritt S1 ist gewährleistet, dass das Verfahren auch wieder Schritt S3 durchläuft, in welchem die Lage der Schicht an eine eventuell neue Lage des Fötus angepasst werden kann.

## Patentansprüche

1. Verfahren zur interaktiven Erfassung von Daten eines Untersuchungsobjekts mittels einer Magnetresonanzanlage (10), wobei das Verfahren folgende Schritte umfasst:
Erfassen der Daten des Untersuchungsobjekts mit der Magnetresonanzanlage (10),
automatisches Rekonstruieren und Darstellen von Bildern anhand der Daten in Echtzeit, **dadurch gekennzeichnet, dass** das Verfahren weiterhin die folgenden Schritte umfasst:
Bestimmen einer Zeitspanne, in welcher eine vorbestimmte Bedingung in den Bildern erfüllt ist,
automatisches Rekonstruieren von Qualitäts-Bildern anhand der Daten, welche innerhalb der Zeitspanne erfasst wurden,
wobei die zeitliche Auflösung bei der Rekonstruktion der Qualitäts-Bilder höher ist als die zeitliche Auflösung bei der Rekonstruktion der Bilder.

2. Verfahren nach Anspruch 1,
**dadurch gekennzeichnet,**
**dass** das Untersuchungsobjekt ein Herz eines lebenden Fötus innerhalb des Körpers seiner Mutter umfasst.

3. Verfahren nach Anspruch 2,
**dadurch gekennzeichnet,**
**dass** die vorbestimmte Bedingung erfüllt ist, wenn keine Bewegung des Fötus innerhalb der Zeitspanne erfasst wird.

4. Verfahren nach einem der vorhergehenden Ansprüche, **dadurch gekennzeichnet,**
**dass** das Erfassen der Daten mittels einer radialen Datenerfassung erfolgt, und
**dass** zwei zeitlich direkt aufeinanderfolgend erfasste radial verlaufende Trajektorien jeweils einen Winkel einschließen, welcher einem goldenen Winkel entspricht.

5. Verfahren nach Anspruch 4,
**dadurch gekennzeichnet,**
**dass** bei der radialen Datenerfassung wiederholt dieselbe vorbestimmte Menge der radial verlaufenden Trajektorien erfasst wird, und
**dass** die Anzahl der Trajektorien der Menge einer hohen Zahl der Fibonacci-Folge entspricht.

6. Verfahren nach einem der Ansprüche 1-3,
**dadurch gekennzeichnet,**
**dass** das Erfassen der Daten mittels einer radialen Datenerfassung erfolgt, und
**dass** die Daten anhand von vorbestimmten Trajektorien erfasst werden, welche eine pseudo-zufällige Verteilung aufweisen.

7. Verfahren nach Anspruch 5 oder 6,
**dadurch gekennzeichnet,**
**dass** das Rekonstruieren der Bilder und/oder Qualitäts-Bilder anhand von für die vorbestimmten Trajektorien vorberechneten Parametern erfolgt.

8. Verfahren nach einem der vorhergehenden Ansprüche,
**dadurch gekennzeichnet,**
**dass** das automatische Rekonstruieren der Qualitäts-Bilder mittels einer K-T-Sparse-SENSE-Rekonstruktion durchgeführt wird.

9. Verfahren nach einem der vorhergehenden Ansprüche,
**dadurch gekennzeichnet,**
**dass** das automatische Rekonstruieren der Qualitäts-Bilder umfasst:
Rekonstruieren von Zwischenbildern aus den Daten,
Ableiten einer Information über eine Herzaktivität des Fötus aus den Zwischenbildern, und
Rekonstruieren der Qualitäts-Bilder abhängig von der Information über die Herzaktivität.

10. Verfahren nach einem der vorhergehenden Ansprüche, **dadurch gekennzeichnet,**
**dass** das Erfassen der Daten ein Erfassen von Daten für eine Schicht innerhalb des Untersuchungsobjekts umfasst,
**dass** mittels der in Echtzeit dargestellten Bilder eine Lage der Schicht überprüft wird,
**dass** abhängig von den in Echtzeit dargestellten Bildern eine Information zur Änderung der Lage der Schicht (4) erfasst wird,
**dass** abhängig von der Information die Lage der Schicht geändert wird,
und
**dass** der Schritt des Erfassens der Daten ein Erfassen von Daten der Schicht (4) in der neuen Lage umfasst.

11. Magnetresonanzanlage (10), die ausgebildet ist, um interaktiv Daten eines Untersuchungsobjekts zu erfassen,
wobei die Magnetresonanzanlage umfasst:
eine HF-Steuereinheit (14), eine Gradientensteuereinheit (15) und eine Bildsequenzsteuerung (16), die ausgebildet sind, um die Daten des Untersuchungsobjekts zu erfassen,
eine Recheneinheit (19), die ausgebildet ist, um Bilder anhand der Daten in Echtzeit zu rekonstruieren,
eine Anzeigeeinheit (18), die ausgebildet ist, um die rekonstruierten Bilder darzustellen, **dadurch gekennzeichnet,**
**dass** die Magnetresonanzanlage weiterhin umfasst:
eine Eingabeeinheit (17), um eine Zeitspanne zu bestimmen, in welcher eine bestimmte Bedingung in den Bildern erfüllt ist, wobei die Recheneinheit zusätzlich ausgestaltet ist, um anhand der Daten, welche innerhalb der Zeitspanne erfasst wurden, Qualitäts-Bilder zu rekonstruieren,
wobei die zeitliche Auflösung bei der Rekonstruktion der Qualitäts-Bilder höher ist als die zeitliche Auflösung bei der Rekonstruktion der Bilder.

12. Magnetresonanzanlage nach Anspruch 11,
**dadurch gekennzeichnet,**
**dass** die Magnetresonanzanlage (10) zur Durchführung des Verfahrens nach einem der Ansprüche 1-10 ausgestaltet ist.

13. Computerprogrammprodukt, welches ein Programm umfasst und direkt in einen Speicher (21) einer programmierbaren Steuerung (20) einer Magnetresonanzanlage (10) ladbar ist, mit Programm-Mitteln, um alle Schritte des Verfahrens nach einem der Ansprüche 1-10 auszuführen, wenn das Programm in der Steuerung (20) der Magnetresonanzanlage (10) ausgeführt wird.

14. Elektronisch lesbarer Datenträger mit darauf gespeicherten elektronisch lesbaren Steuerinformationen, welche derart ausgestaltet sind, dass sie bei Verwendung des Datenträgers in einer Steuerung (20) einer Magnetresonanzanlage (10) das Verfahren nach einem der Ansprüche 1-10 durchführen.

## Claims

1. Method for the interactive acquisition of data from an object under investigation by means of a magnetic resonance system (10),
wherein the method comprises the following steps:
acquiring the data from the object under investigation using the magnetic resonance system (10),
automatically reconstructing and displaying images on the basis of the data in real time, **characterised in that** the method furthermore comprises the following steps:
determining a time interval, during which a predetermined condition in the images is met,
automatically reconstructing quality MR images on the basis of the data which was acquired within the time interval,
wherein the temporal resolution during reconstruction of the quality MR images is higher than the temporal resolution during reconstruction of the MR images.

2. Method according to claim 1,
**characterised in that**
the object under investigation comprises a heart of a living fetus within the body of its mother.

3. Method according to claim 2,
**characterised in that**
the predetermined condition is met if no movement of the fetus is acquired within the time interval.

4. Method according to one of the preceding claims,
**characterised in that**
the data is acquired by means of radial data acquisition, and
two temporally directly successively acquired radially extending trajectories in each case form an angle which corresponds to a golden angle.

5. Method according to claim 4,
**characterised in that**
the same predetermined set of radially extending trajectories are repeatedly acquired during radial data acquisition, and the number of trajectories of the set corresponds to a high number in the Fibonacci sequence.

6. Method according to one of claims 1-3,
**characterised in that**
the data is acquired by means of radial data acquisition, and the data is acquired on the basis of predetermined trajectories which have a pseudo-random distribution.

7. Method according to claim 5 or 6,
**characterised in that**
the images and/or quality images are reconstructed on the basis of parameters which are precalculated for the predetermined trajectories.

8. Method according to one of the preceding claims,
**characterised in that**
the quality images are automatically reconstructed by means of a K-T sparse SENSE reconstruction.

9. Method according to one of the preceding claims,
**characterised in that**
automatic reconstruction of the quality images comprises:
reconstructing intermediate images from the data,
deriving information about the cardiac activity of the fetus from the intermediate images, and
reconstructing the quality images depending on the information about cardiac activity.

10. Method according to one of the preceding claims, **characterised in that**
acquiring the data comprises acquiring data for a slice within the object under investigation,
a position of the slice is checked by means of the images displayed in real time,
information for modifying the position of the slice (4) is acquired depending on the images displayed in real time,
the position of the slice is modified depending on the information,
and
the step of acquiring the data comprises acquiring data from the slice (4) in the new position.

11. Magnetic resonance system (10) which is configured to interactively acquire data from an object under investigation, wherein the magnetic resonance system comprises:
an HF control unit (14), a gradient control unit (15) and an image sequence controller (16) which are configured to acquire the data from the object under investigation,
a computing unit (19) which is configured to reconstruct images in real time on the basis of the data,
a display unit (18) which is configured to display the reconstructed images, **characterised in that** the magnetic resonance system furthermore comprises:
an input unit (17) for determining a time interval in which a specific condition is met in the images,
wherein the computing unit is additionally designed to reconstruct quality images on the basis of the data which was acquired within the time interval,
wherein the temporal resolution during reconstruction of the quality MR images is higher than the temporal resolution during reconstruction of the MR images.

12. Magnetic resonance system according to claim 11,
**characterised in that**
the magnetic resonance system (10) is designed to carry out the method according to one of claims 1-10.

13. Computer program product which comprises a program and can be loaded directly into a memory (21) of a programmable controller (20) of a magnetic resonance system (10), having program means for carrying out all the steps of the method according to one of claims 1-10 when the program is executed in the controller (20) of the magnetic resonance system (10).

14. Electronically readable data storage medium with electronically readable control information stored thereon, which information is designed such that, on using the data storage medium in a controller (20) of a magnetic resonance system (10), it carries out the method according to one of claims 1-10.

## Revendications

1. Procédé de détection interactive de données d'un objet à examiner, au moyen d'une installation (10) de résonnance magnétique, dans lequel le procédé comprend les stades suivants :
détection des données de l'objet à examiner par l'installation (10) de résonnance magnétique,
reconstruction automatique et représentation d'images à l'aide des données en temps réel, **caractérisé en ce que** le procédé comprend en outre les stades suivants :
définition d'un laps de temps, dans lequel une condition définie à l'avance est satisfaite dans les images,
reconstruction automatique d'images de qualité à l'aide des données, qui ont été détections dans le laps de temps,
dans lequel la résolution temporelle, lors de la reconstruction des images de qualité est plus grande que la résolution temporelle, lors de la reconstruction des images.

2. Procédé suivant la revendication 1,
**caractérisé en ce que**
l'objet à examiner comprend un coeur d'un foetus vivant dans le corps de sa mère.

3. Procédé suivant la revendication 2,
**caractérisé en ce que**
la condition définie à l'avance est satisfaite s'il n'a pas été détecté de mouvement du foetus dans le laps de temps.

4. Procédé suivant l'une des revendications précédentes,
**caractérisé en ce que**
la détection des données s'effectue au moins au moyen d'une détection de données radiale, et
**en ce que** deux trajectoires, s'étendant radialement, détectées en se suivant directement dans le temps, font respectivement un angle, qui correspondant à un angle d'or.

5. Procédé suivant la revendication 4,
**caractérisé en ce que**
lors de la détection de données radiale, on détecte, de manière répétée, la même quantité définie à l'avance des trajectoires s'étendant radialement, et
le nombre des trajectoires correspond à un nombre haut de la suite de Fibonacci.

6. Procédé suivant l'une des revendications 1 à 3, **caractérisé en ce que**
la détection des données s'effectue au moyen d'une détection de données radiale, et
**en ce que** l'on détecte les données à l'aide de trajectoires définies, qui ont une répartition pseudo aléatoire.

7. Procédé suivant la revendication 5 ou 6,
**caractérisé en ce que**
la reconstruction des images et/ou des images de qualité s'effectue à l'aide de paramètres calculés à l'avance pour les trajectoires définies à l'avance.

8. Procédé suivant l'une des revendications précédentes,
**caractérisé en ce que**
l'on effectue la reconstruction automatique des images de qualité au moyen d'une reconstruction K-T-Sparse-SENSE.

9. Procédé suivant l'une des revendications précédentes, **caractérisé en ce que**
la reconstruction automatique des images de qualité comprend :
la reconstruction d'images intermédiaires à partir des données,
la déduction d'une information sur une activité cardiaque du foetus à partir des images intermédiaires,
et la reconstruction des images de qualité en fonction de l'information sur l'activité cardiaque.

10. Procédé suivant l'une des revendications précédentes, **caractérisé en ce que**
la détection des données comprend une détection de données pour une couche au sein de l'objet à examiner,
**en ce que** l'on contrôle au moyen des images représentées en temps réel, une position de la couche,
en e que l'on détecte, en fonction des images représentées en temps réel, une information de modification de la position de la couche (4),
**en ce que** l'on modifie la position de la couche en fonction de l'information, et
**en ce que** le stade de la détection des données comprend une détection de données de la couche (4) dans la nouvelle position.

11. Installation (10) de résonnance magnétique, qui est constituée pour détecter interactivement des données d'un objet à examiner,
dans laquelle l'installation de résonnance magnétique comprend :
une unité (14) de commande HF, une unité (15) de commande de gradient et une commande (16) de séquence d'images, qui sont constituées pour détecter les données de l'objet à examiner,
une unité (19) de calcul, qui est constituée pour reconstruire en temps réel des images à l'aide des données,
une unité (18) d'affichage, qui est constituée pour représenter les images reconstruites, **caractérisée en ce que** l'installation de résonnance magnétique comprend en outre :
une unité (17) d'entrée pour définir un laps de temps, dans lequel une condition définie est satisfaite dans les images, l'unité de calcul étant conformée en outre pour reconstruire des images de qualité, à l'aide des données, qui ont été détectées dans le laps de temps,
dans laquelle la résolution temporelle, lors de la reconstruction des images de qualité, est plus grande que la résolution temporelle, lors de la reconstruction des images.

12. Installation de résonnance magnétique suivant la revendication 11,
**caractérisée en ce que**
l'installation (10) de résonnance magnétique est conformée pour effectuer le procédé suivant l'une des revendications 1 à 10.

13. Produit de programme d'ordinateur, qui comprend un programme et qui peut être chargé directement dans une mémoire (21) d'une commande (20) programmable d'une installation (10) de résonnance magnétique, comprenant des moyens de programme pour effectuer tous les stades du procédé suivant l'une des revendications 1 à 10, lorsque le programme est exécuté dans la commande (20) de l'installation (10) de résonnance magnétique.

14. Support de données, déchiffrable électroniquement, sur lequel sont mises en mémoire des informations de commande pouvant être déchiffrées électroniquement, qui sont conformées, de manière à ce qu'elles effectuent, lors de l'utilisation du support de données dans une commande (20) d'une installation (10) de résonnance magnétique, le procédé suivant l'une des revendications 1 à 10.
